# EUROPEAN PATENT APPLICATION

(11) **EP 3 620 111 A1**
(43) Date of publication of application: **11.03.2020**
(21) Application number: 19187840.4
(22) Date of filing: 23.07.2019
(51) Int. Cl.: A61B 6/00, A61B 8/00, A61B 8/08, A61N 5/00, A61N 7/00, G01S 7/52, G06T 5/50, A61B 5/00, A61B 17/00, A61B 90/00, A61N 5/10

(54) **POSITION MEASUREMENT DEVICE, TREATMENT SYSTEM INCLUDING THE SAME, AND POSITION MEASUREMENT METHOD**

(30) Priority: 04.09.2018 JP 2018165478
(71) Applicant: Hitachi, Ltd., Tokyo 100-8280 (JP)
(72) Inventor: SASAKI, Kota, Tokyo, 100-8280 (JP); MIZOTA, Hirohisa, Tokyo, 100-8280 (JP); KOURAI, Yuusuke, Tokyo, 100-8280 (JP)
(74) Representative: MERH-IP Matias Erny Reichl Hoffmann Patentanwälte PartG mbB

(57) **Abstract**

To provide a position measurement device capable of measuring a target position with high accuracy according to an ultrasonic image during treatment, a treatment system including the device, and a position measurement method. A first image is constructed according to an ultrasonic waveform acquired by an ultrasonic sensor 104C, a three-dimensional image acquired in advance and the first image are collated on the basis of sensor position information acquired by a sensor position measurement unit 105 to calculate sound velocities of each body tissue of a patient 100, a second image is constructed according to the ultrasonic waveform acquired by the ultrasonic sensor 104C using the calculated sound velocities of each body tissue of the patient 100, and a target tissue position is calculated according to the second image.

## Description

### BACKGROUND OF THE INVENTION

The present invention relates to a position measurement device for measuring a three-dimensional position of a specific target in a patient by ultrasonic waves, a treatment system including the device, and a position measurement method.

Patent Literature 1 (JP-A-2003-117010) provides an example of a non-invasive and highly safe radiation system capable of detecting a movement of a treatment target site caused by respiration, pulsation, or body motion of a patient and performing highly accurate radiation irradiation without increasing in size and undue complication of the device itself, and an example of a program used for operating the device, and a computer readable record medium recording the program. Patent Literature 1 discloses that an ultrasonic image for treatment planning is taken simultaneously as a CT image for treatment planning is taken, at the time of treatment, a real-time imaged ultrasonic image for treatment and an ultrasonic image for treatment planning are compared, it is determined whether a correlation value of both ultrasonic images is equal to or larger than a predetermined value, and a radiation irradiation means is controlled to perform irradiation for the treatment target site only when this correlation value is equal to or larger than the predetermined value.

To accurately align an ultrasonic image with a medical image taken by a medical image diagnostic device of the different kind from an ultrasonic diagnostic device, Patent Literature 2 (JP-A-2012-075747) discloses that an image processing unit of an ultrasonic diagnostic device includes a pseudo ultrasonic image forming unit, an index computing unit, and an aligning unit, wherein the pseudo ultrasonic image forming unit forms a pseudo ultrasonic image by converting a three-dimensional medical image in a pseudo manner into an ultrasonic image based on physical characteristics of each tissue depicted in the three-dimensional medical image used as an alignment object of the ultrasonic image and sound source information, the index computing unit computes an index showing similarity between the pseudo ultrasonic image and the ultrasonic image, and the aligning unit repeatedly carries out pseudo ultrasonic image forming processing and index computing processing by changing the sound source information and carries out alignment based on a position in the three-dimensional medical image of the pseudo ultrasonic image in which the index is optimal.

In cancer radiation, in order to efficiently perform the treatment, it is important that a region to be irradiated with the radiation and a treatment target region where cancer tumor exists are accurately matched with each other.

One method of measuring the tumor position in the body is a method using ultrasonic waves as described in Patent Literature 1 and Patent Literature 2.

In a related radiation system, a position of a body tissue which is a treatment target site is specified according to information of a computed tomography (CT) image acquired in advance, and treatment planning is set on the basis of the specified position. According to the treatment planning, a patient is fixed to a treatment table of the radiation system, and radiation is irradiated to a body tissue which is a treatment target site of the patient by controlling characteristics of the radiation such as an irradiation direction and an intensity to perform treatment.

However, it is known that the treatment target site of the patient moves from a planned radiation irradiation position due to the respiration of the patient during the radiation irradiation, and therefore, performing treatment in high accuracy has been a problem to be solved.

To solve this problem, a high accuracy treatment method is established in which a marker composed of gold and the like is embedded in advance in a patient body, this marker is imaged and tracked by an X-ray transmission image to detect the movement of the treatment target site, and the marker is used in the treatment planning set in advance and radiation control during applying irradiation.

Meanwhile, it is desired to realize an inner-body position measurement device which can accurately depict soft tissue while reducing exposure dose of X-ray radiation during treatment and can cope with the movement of the patient due to respiration. There is a method using an ultrasonic image can be used to realize this purpose.

Instead of using the marker and the X-ray, Patent Literature 1 discloses that radiation is irradiated at a timing at which the correlation value between the ultrasonic image acquired at the same time as the CT image which is acquired in advance, and the ultrasonic image acquired during treatment is high.

Here, a sound velocity of the ultrasonic wave propagating through the body varies depending on different tissue. For example, it is known that the sound velocity in fat is about 1450 m/s, the sound velocity in blood/muscle/organ is about 1530 to 1630 m/s, and the sound velocity in bone is about 2700 to 4100 m/s. Further, the ultrasonic waves propagating through medium having different sound velocities undergo refraction according to Snell's law.

In addition, since a state of the body tissue differs for each patient, the sound velocity is different for different patients even in the same tissue.

For this reason, in the method of constructing an ultrasonic image which regards the human tissue as uniform as in the above-mentioned Patent Literature 1, the ultrasonic image is distorted due to existence of the difference of the sound velocity between different tissue and the refraction according to Snell's law. Therefore, the correlation value with the CT image decreases, and an error may occur between an estimated position of the tumor at the calculated irradiation timing and an actual position of the tumor.

Patent Literature 2 describes a method in which the image processing device sets a virtual sound source on the three-dimensional medical image such as a CT image or a magnetic resonance imaging (MRI) image, generates a pseudo ultrasonic image corresponding to the three-dimensional medical image, and calculates the sound velocity by collating the three-dimensional medical image with the pseudo ultrasonic image.

However, the method described in Patent Literature 2 does not calculate the sound velocity on the basis of measured ultrasonic waveform data. Therefore, there is a problem that an error may occur between an actual position of the tumor and a computed position.

### SUMMARY OF THE INVENTION

The invention has been made in view of the above problems, and an object of the invention is to provide a position measurement device capable of measuring a target position with high accuracy according to an ultrasonic image during treatment, a treatment system including the device, and a position measurement method.

The invention includes a plurality of means for solving the above-mentioned problems, and one example thereof is a position measurement device configured to measure a position of a body tissue of a patient by ultrasonic waves, and the position measurement device includes an ultrasonic sensor; a sensor position measurement unit configured to measure a position of the ultrasonic sensor; a position calculation device configured to construct an ultrasonic image according to an ultrasonic waveform acquired by the ultrasonic sensor; wherein the position calculation device is configured to: construct a first image according to the ultrasonic waveform acquired by the ultrasonic sensor, collate a three-dimensional image acquired in advance with the first image on the basis of sensor position information acquired by the sensor position measurement unit to calculate sound velocities of each body tissue of the patient, construct a second image according to the ultrasonic waveform acquired by the ultrasonic sensor using the calculated sound velocities of each body tissue of the patient, and calculate a position of a target tissue according to the second image.

According to the invention, a target position can be measured with high accuracy according to an ultrasonic image during treatment. Problems, configurations, and effects other than those described above will be further clarified with the following description of embodiments.

### BRIEF DESCRIPTION OF THE DRAWINGS

FIG. 1 is a functional block diagram of a position measurement device according to a first embodiment of the invention.
FIG. 2A is a conceptual diagram of imaging a human body by ultrasonic waves.
FIG. 2B is a cross-sectional view of FIG. 2A.
FIG. 2C is a diagram showing an example of an ultrasonic image obtained by a system shown in FIG. 2B.
FIG. 3A is a conceptual diagram of a three-dimensional medical image obtained by the position measurement device according to the first embodiment.
FIG. 3B is a conceptual diagram of an ultrasonic image obtained by the position measurement device according to the first embodiment.
FIG. 4 is a conceptual diagram of a correcting method for an ultrasonic image obtained by the position measurement device according to the first embodiment.
FIG. 5A is a conceptual diagram of a sound velocity database used for comparison.
FIG. 5B is a conceptual diagram of a sound velocity database used by the position measurement device according to the first embodiment.
FIG. 6 is a flowchart showing an example of image collation processing executed by the position measurement device according to the first embodiment.
FIG. 7 is a flowchart showing an example of sound velocity calculation processing executed by the position measurement device according to the first embodiment.
FIG. 8 is a flowchart showing an example of ultrasonic image construction processing executed by the position measurement device according to the first embodiment.
FIG. 9 is a conceptual diagram of a radiation system including a position measurement device according to a second embodiment of the invention.
FIG. 10 is a conceptual diagram of an ultrasonic treatment system including a position measurement device according to a third embodiment of the invention.
FIG. 11 is a conceptual diagram of a position measurement device according to a fourth embodiment of the invention.
FIG. 12 is a conceptual diagram of a position measurement device according to a fifth embodiment of the invention.
FIG. 13 is a flowchart showing an example of a reconstruction method for an ultrasonic image obtained by the position measurement device according to the fifth embodiment.

### DESCRIPTION OF EMBODIMENTS

Hereinafter, embodiments of a position measurement device, a treatment system including the device, and a position measurement method of the invention will be described with reference to drawings.

### <First Embodiment>

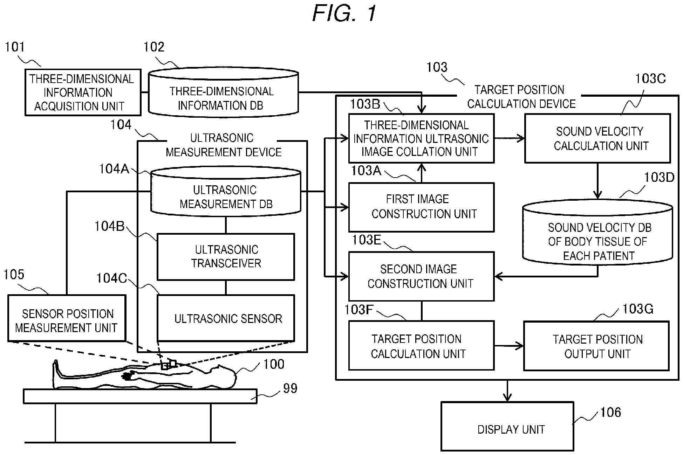

A position measurement device and a position measurement method according to a first embodiment of the invention will be described with reference to FIGS. 1 to 8. In FIGS. 1 to 8, parts in one figure identical to those in other figures are denoted by the same reference numerals.

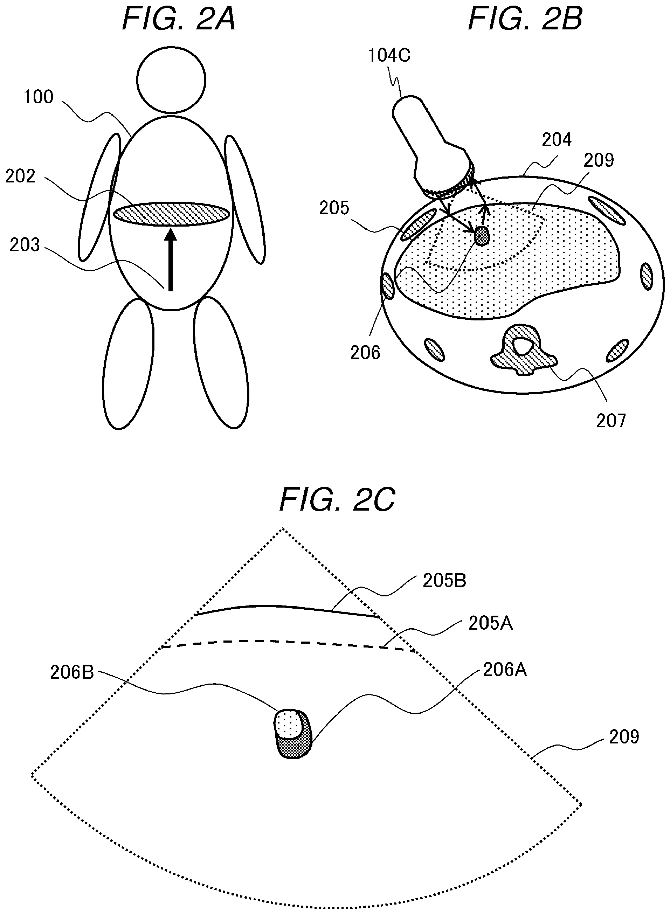

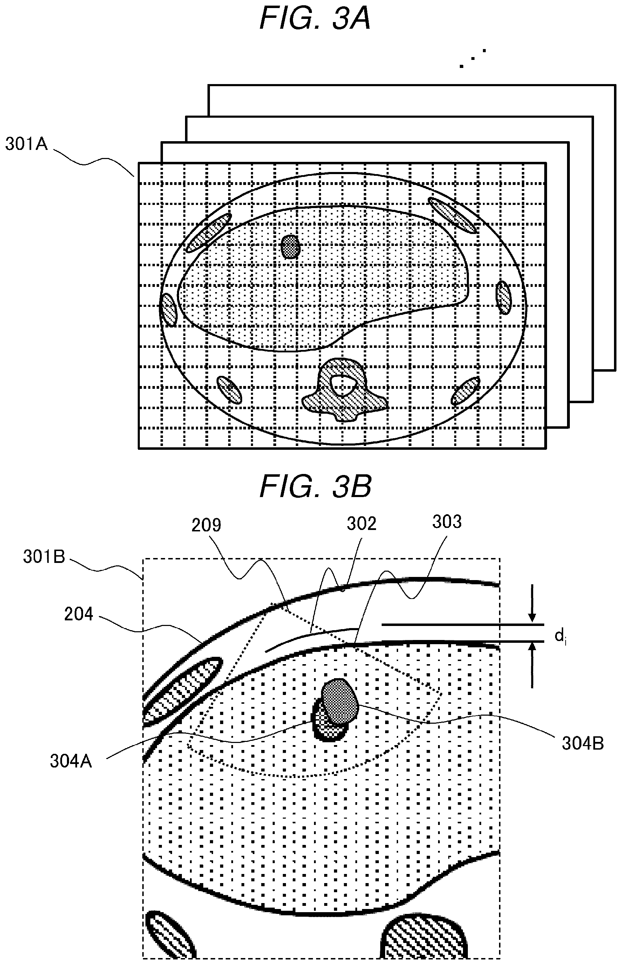

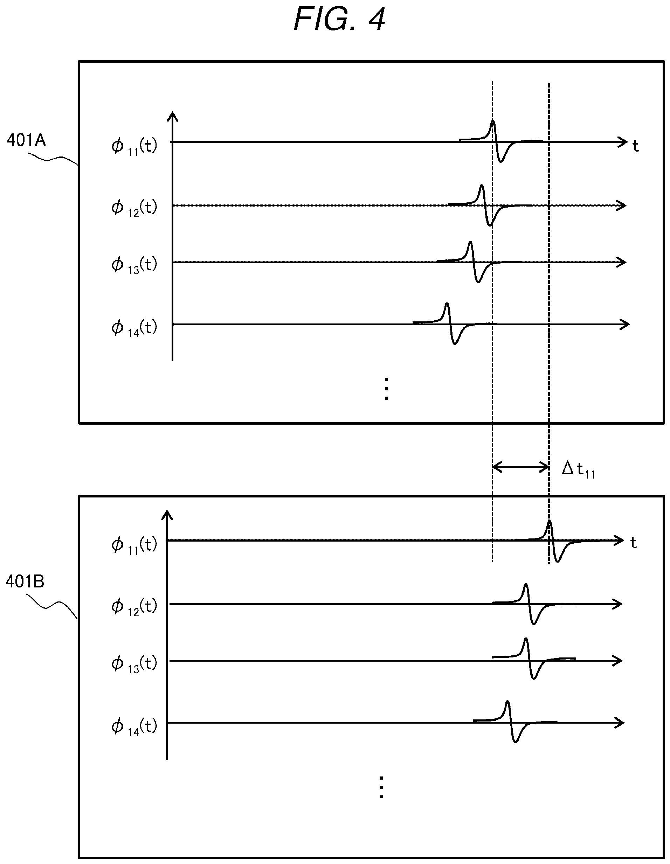

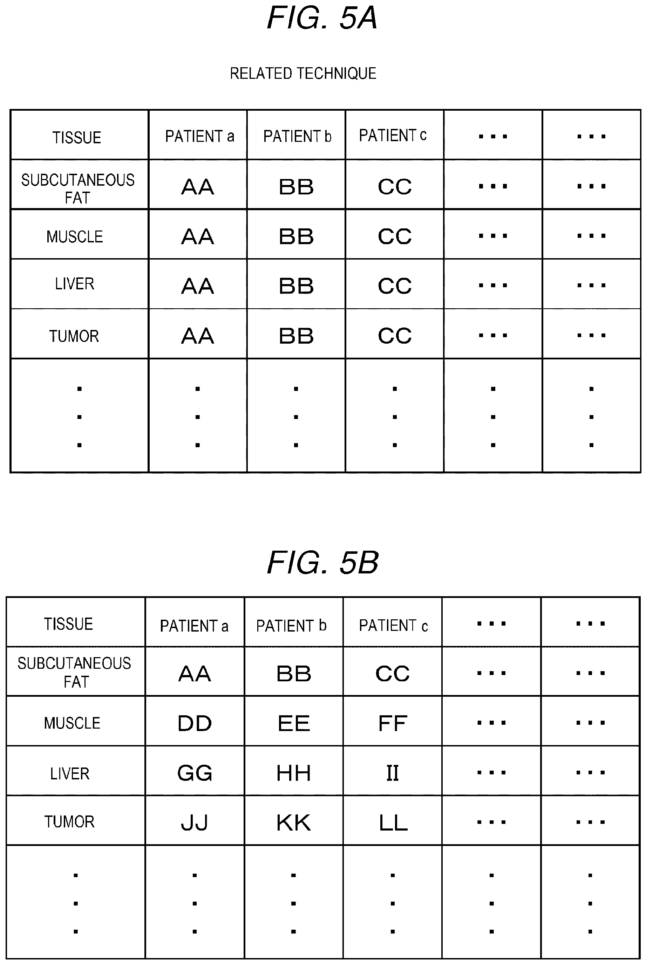

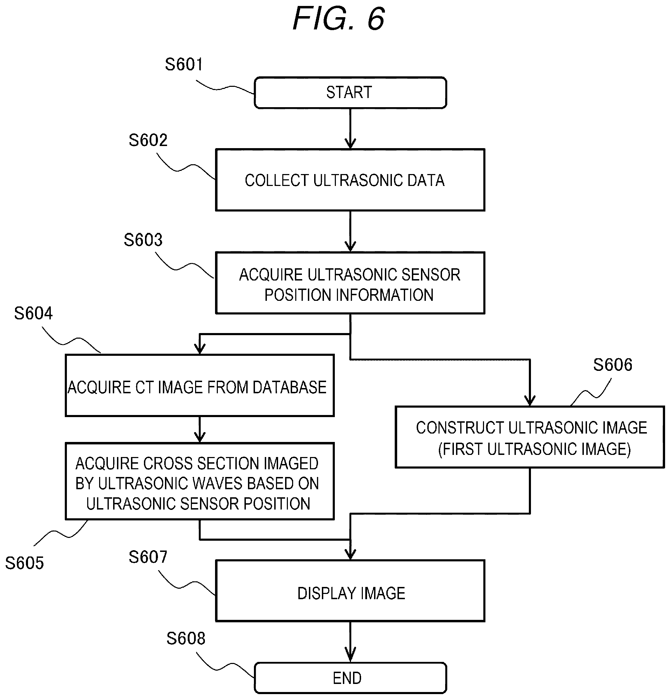

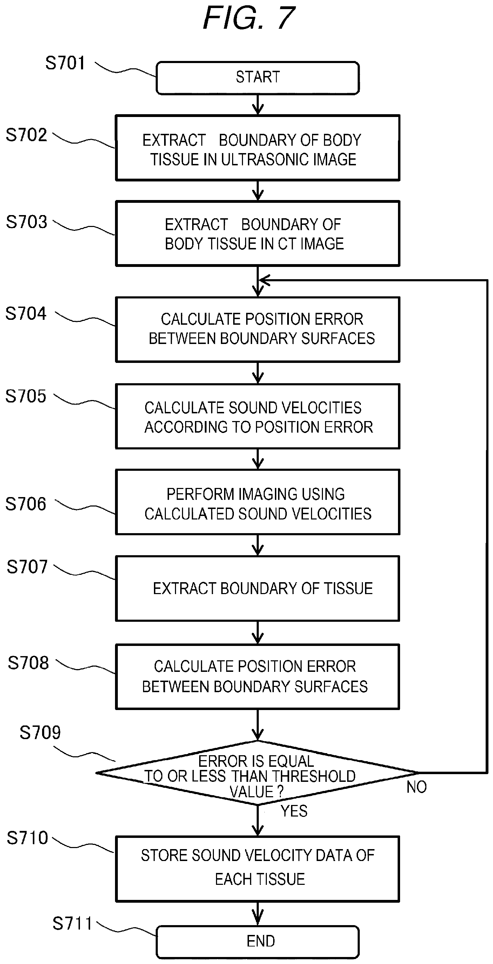

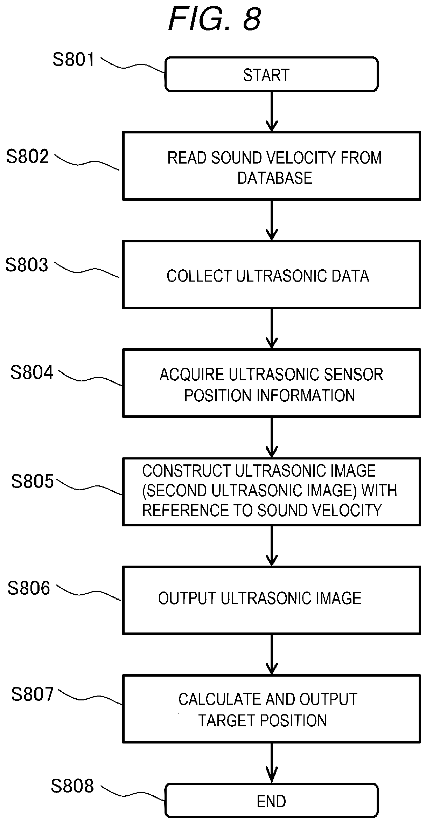

FIG. 1 is a conceptual view of the position measurement device according to the present embodiment. FIGS. 2A to 2C are schematic views of a patient body and ultrasonic images obtained by imaging the same. FIGS. 3A and 3B are conceptual diagrams showing a sound velocity calculation method based on comparing of a cross-sectional view of three-dimensional information of the patient with an ultrasonic image. FIG. 4 is a conceptual view showing time domain data of ultrasonic reception data in the sound velocity calculation method. FIG. 5B is a database holding sound velocity information calculated according to the present embodiment. FIG. 6 is a flowchart showing image collation processing for the three-dimensional information and the ultrasonic image according to the present embodiment. FIG. 7 is a flowchart showing sound velocity calculation processing according to the present embodiment. FIG. 8 is a flowchart showing ultrasonic image construction processing with reference to sound velocity data in the sound velocity database according to the present embodiment.

First, a configuration and a function of the position measurement device according to the present embodiment will be described with reference to FIGS. 1 to 5B.

The position measurement device shown in FIG. 1 is a device that measures, by ultrasonic waves, a three-dimensional target position of a body tissue of a patient 100 who is fixed on a bed.

As shown in FIG. 1, the position measurement device includes a three-dimensional information acquisition unit 101, a three-dimensional information database 102, a target position calculation device 103, an ultrasonic measurement device 104, a sensor position measurement unit 105, and a display unit 106.

In the position measurement device, the patient 100 is fixed to a bed 99 in a state where an ultrasonic sensor 104C whose position is fixed by a fixing jig (not shown) such as a robot arm is disposed so as to press against a body surface.

The ultrasonic measurement device 104 includes an ultrasonic measurement database 104A, an ultrasonic transceiver 104B, and the ultrasonic sensor 104C.

The ultrasonic sensor 104C receives an electric signal from the ultrasonic transceiver 104B, excites ultrasonic waves, and transmits the ultrasonic waves to the body of the patient 100. Further, the ultrasonic sensor 104C receives the reflected ultrasonic waves scattering in the body of the patient 100, converts the received ultrasonic waves into an electric signal, and transmits the converted electric signal to the ultrasonic transceiver 104B.

The ultrasonic transceiver 104B amplifies the electric signal received from the ultrasonic sensor 104C and transmits the amplified signal to the ultrasonic measurement database 104A. The ultrasonic measurement database 104A stores the received ultrasonic reception signal.

Elements that mutually converts the electric signal and the ultrasonic waves into each other is disposed inside the ultrasonic sensor 104C, and the ultrasonic transceiver 104B controls the excitation timing of each element so that a focus position of the ultrasonic waves can be scanned.

The ultrasonic reception signal due to reflection and scattering held in the ultrasonic measurement database 104A is transmitted to the target position calculation device 103, and is synthesized by a first image construction unit 103A and a second image construction unit 103E, and thus the ultrasonic image in an ultrasonic operation range can be acquired.

FIGS. 2A to 2C are conceptual diagrams showing how the patient body is imaged by the ultrasonic waves.

A cross-sectional view of a certain area 202 in the human body of the patient 100 shown in FIG 2A as viewed from a patient foot side (image checking direction 203) is shown in FIG. 2B. As shown in FIG. 2B, for example, a body surface 204, an organ 205, a tumor 206, a bone 207, and the like are included in the cross-sectional view of the patient 100.

In the cross section, a region 209 is an example of a region in the body to be imaged by the ultrasonic sensor 104C disposed in the body surface 204, and the ultrasonic image formed according to the ultrasonic signal is as shown in FIG. 2C.

Here, the sound velocity in different body tissues of the patient 100 is different. In addition, for the patient 100, even in the same tissue, there are different sound velocities.

Further, a case where acoustic impedances are different between tissues in the body is taken into consideration. In this case, according to Snell's law, refraction occurs once a sound passes a boundary, and, for example, propagates along a path as shown in FIG. 2B. Therefore, the signal received by each element of the ultrasonic sensor has a deviation in propagation time t as compared with a case where the refraction is not taken into account. For this reason, for example, in the ultrasonic image shown in FIG. 2C, a boundary 205B and a target position 206B of the body tissue which are confirmed on the ultrasonic image deviate from an actual boundary position 205A and an actual target position 206A, respectively.

Therefore, according to the invention, three-dimensional movement information on the body tissue and the target of the patient 100 is acquired and stored in advance. The stored three-dimensional information and the ultrasonic image are collated, and the sound velocity is calculated for each patient 100 and each body tissue, and the calculated results are stored in the sound velocity database 103D.

Then, in constructing of the ultrasonic image when the target position is actually measured, the deviation of the target actual position in the ultrasonic image is corrected by reflecting the sound velocities of each body tissue of the patient 100 stored in the sound velocity database 103D in advance, thereby the target position can be measured with high accuracy.

In FIG. 1, the three-dimensional information acquisition unit 101 captures a three-dimensional image such as a CT image or an MRI image at least including a target whose position to be calculated, for example, at a timing synchronized with respiration.

At this time, the ultrasonic sensor 104C may generate artifacts which influence the three-dimensional image. In such a case, it is desirable to use a dummy sensor that simulates pressing of the body surface by the ultrasonic sensor 104C and has few artifacts, instead of the ultrasonic sensor 104C used in acquiring of the three-dimensional information.

The acquired information is transmitted to the three-dimensional information database 102 and stored therein.

The sensor position measurement unit 105 is, for example, an optical sensor, a magnetic sensor, an ultrasonic sensor, and the like, and is a device which measures an actual position at which the ultrasonic sensor 104C is disposed during transmission or reception of the ultrasonic waves in synchronization with acquisition of the ultrasonic signal.

Various other methods other than the optical sensor and the like can be adopted as the sensor position measurement unit 105, and accordingly, the example described here is not intended to limit the embodiments of the invention.

Information on a sensor range of the ultrasonic sensor 104C, that is, information on a range of a comparison region between the actual ultrasonic image and the two-dimensional cross-sectional image obtained according to the three-dimensional information stored in the three-dimensional information database 102 is stored in the ultrasonic measurement database 104A.

The target position calculation device 103 constructs the ultrasonic image according to an ultrasonic waveform acquired by the ultrasonic sensor 104C to calculate a three-dimensional position of a target body tissue in the patient 100.

The target position calculation device 103 includes the first image construction unit 103A, a three-dimensional information ultrasonic image collation unit 103B, a sound velocity calculation unit 103C, a sound velocity database 103D with respect to body tissue of each patient, the second image construction unit 103E, a target position calculation unit 103F, and a target position output unit 103G.

The first image construction unit 103A constructs an ultrasonic image (first image) according to the ultrasonic reception signal stored in the ultrasonic measurement database 104A. The constructed ultrasonic image is output to the three-dimensional information ultrasonic image collation unit 103B.

The three-dimensional information ultrasonic image collation unit 103B acquires sensor position information measured by the sensor position measurement unit 105 from the ultrasonic measurement database 104A, and determines which cross-sectional image (two-dimensional cross-sectional image) is to be obtained from the three-dimensional information stored in the three-dimensional information database 102. Then, by referring to range information of the comparison region with respect to the three-dimensional information stored in the ultrasonic measurement database 104A, both images of the determined two-dimensional cross-sectional image and the ultrasonic image output from the first image construction unit 103A are collated.

Before the collation, it is desirable to match resolutions of the ultrasonic image output from the first image construction unit 103A and the determined two-dimensional cross-sectional image by adjusting imaging areas, pixel sizes, and luminance of both images.

For example, the three-dimensional information ultrasonic image collation unit 103B first compares the resolution of the three-dimensional information image stored in the three-dimensional information database 102 with the resolution of the actual ultrasonic image, and interpolates pixel data for either one of the three-dimensional information image and the actual ultrasonic image to match the resolutions of both images.

As a method of interpolating pixels, nearest neighbor interpolation, bilinear interpolation, bicubic interpolation, and the like are generally known, and an appropriate method can be selected according to the target position calculation accuracy. Although the image to be subjected to the interpolation processing may be either image, it is desirable to match an image with a lower resolution to an image with a higher resolution.

In the case of matching the resolutions of both images, it is desirable that the three-dimensional information ultrasonic image collation unit 103B realizes matching by using feature points in both images.

The sound velocity calculation unit 103C calculates the sound velocities of each body tissue of the patient 100 according to the two-dimensional cross-sectional image based on the determined three-dimensional information and the ultrasonic image output from the first image construction unit 103A which are collated by the three-dimensional information ultrasonic image collation unit 103B. Information on the calculated sound velocities of each body tissue is output to the sound velocity database 103D.

An example of a method of calculating the sound velocities of the body tissue by collation of the ultrasonic image and the three-dimensional information will be described with reference to FIGS. 3A and 3B.

The three-dimensional information ultrasonic image collation unit 103B specifies a region corresponding to the ultrasonic image 209, which is constructed by the first image construction unit 103A as shown in FIG. 3B from the two-dimensional cross-sectional image 301A in a specific cross section of the three-dimensional information as shown in FIG. 3A, on the basis of, for example, information on coordinates and an inclination angle of the ultrasonic sensor of the sensor position measurement unit 105. At this time, the ultrasonic image is constructed using a sound velocity v₀ of the ultrasonic waves in the body, which has been previously set and reported in the literature and the like.

An example of the comparison between the specified two-dimensional cross-sectional image 301B and the ultrasonic image is shown in FIG. 3B. The sound velocity calculation unit 103C compares a position Rᵢ of a boundary surface 303 of the body tissue depicted on the cross section specified from the three-dimensional information with a position rᵢ of a boundary surface 302 of the body tissue depicted on the ultrasonic image to calculate a position error dᵢ therebetween. In the sound velocity calculation unit 103C, a position of a boundary surface of a target 304A depicted on the cross section specified from the three-dimensional information and a position of a boundary surface of a target 304B depicted on the ultrasonic image can be used.

An accurate sound velocity v in the body tissue is expressed as v = v₀ + v_{ci} in which v_{ci} = v₀ (dᵢ/rᵢ) using the sound velocity correction term v_{c}. The sound velocity calculation unit 103C calculates the sound velocities of each body tissue by correcting the sound velocity calculation with one or more indices in both images.

It is desirable that the sound velocity calculation unit 103C reconstructs the first image according to the calculated sound velocities, and continuously calculates the sound velocities and constructs the reconstructed image until a difference between the three-dimensional image acquired in advance and the reconstructed first image is equal to or less than a predetermined value. The sound velocity calculation unit 103C stores the sound velocities obtained when the difference is equal to or less than the predetermined value in the sound velocity database 103D as the sound velocities of each body tissue of the patient.

Indices to be collated are, for example, a center of gravity of the body tissue, a relative distance between a plurality of tissues, and the like, and it is desirable to use a value by which the position information of both images can be compared.

A method of correcting time domain data of the ultrasonic reception signal using the sound velocities calculated as described above will be described with reference to FIG. 4. In FIG. 4, Φₙₘ is a signal intensity at which an n-th element receives the signal transmitted from an m-th element on the ultrasonic sensor 104C.

As shown in FIG. 4, between a reception signal 401A before calculating the sound velocities of each tissue and a reception signal 401B after calculating the sound velocities, there is a propagation time difference Δtₙₘ for the same reception signal Φₙₘ.

Therefore, by reconstructing the ultrasonic image also after the sound velocities of each tissue are calculated, the time difference Δtₙₘ is corrected for the reception signals Φₙₘ of all the ultrasonic waves, and an image closer to an actual state of the body tissue can be obtained.

The sound velocity database 103D stores the sound velocities of each body tissue of the patient 100 calculated by the target position calculation device 103.

FIG. 5B shows an example of the calculated sound velocity data stored in the sound velocity database 103D.

In related ultrasonic examinations, the image is constructed by sound velocities, for example, data in which the sound velocities of each body tissue for each patient are uniform. In contrast, in the present embodiment, as shown in FIG. 5B, the calculated sound velocity data for each body tissue and each patient is stored in the sound velocity database 103D.

The target position calculation device 103 calculates the actual three-dimensional position of a target body tissue. Hereinafter, a configuration in which a second image is constructed according to the ultrasonic waveform acquired by the ultrasonic sensor 104C using the calculated sound velocities for each body tissue of the patient 100 and a position of a target tissue is calculated according to the second image will be described.

The second image construction unit 103E reads the sound velocity data held in the sound velocity database 103D. Further, the ultrasonic image (second image) is constructed on the basis of the ultrasonic reception signal received by the ultrasonic sensor 104C and stored in the ultrasonic measurement database 104A, the position information of the ultrasonic sensor 104C measured by the sensor position measurement unit 105, and the sound velocity data when the actual three-dimensional position of the target body tissue is calculated.

The target position calculation unit 103F calculates the three-dimensional position of the target body tissue according to the ultrasonic image constructed by the second image construction unit 103E, and outputs target position coordinates to the target position output unit 103G.

The target position output unit 103G converts the input three-dimensional position into a display signal and outputs the display signal to the display unit 106 for display.

Output methods include, for example, a method in which a relative position based on reference coordinates is displayed numerically on a monitor, a method in which a position corresponding to the acquired three-dimensional information is displayed on a monitor or a method in which the three-dimensional information is transmitted wiredly or wirelessly as an appropriately encoded electrical signal. In addition, the output method can employ various methods depending on the purpose of use of the calculated body tissue position, and therefore, the examples described here are not intended to limit the embodiments of the present invention.

The first image construction unit 103A, the three-dimensional information ultrasonic image collation unit 103B, the sound velocity calculation unit 103C, the second image construction unit 103E, the target position calculation unit 103F, and the target position output unit 103G in the target position calculation device 103, and the ultrasonic transceiver 104B of the ultrasonic measurement device 104 can be realized by causing a computer or a Field-Programmable Gate Array (FPGA) including a CPU, a memory, an interface, and the like to read programs to execute the calculation. These programs are stored in an internal storage medium or an external recording medium (not shown) in each configuration, and read and executed by the CPU.

The control processing of the operation may be integrated into one program, or may be divided into a plurality of programs or a combination thereof. A part or all of the programs may be realized by dedicated hardware, or may be modularized. Further, the various programs may be installed in each device such as a program distribution server, an internal storage medium, or an external storage medium.

In addition, programs are not necessary to be independent of each other, and two or more of them may be integrated and made in common to only share the processing. In addition, at least some of the configurations may be connected via a wired or wireless network. The same applies to the embodiments to be described below.

The three-dimensional information database 102, the ultrasonic measurement database 104A, and the sound velocity database 103D can be configured using a memory, a hard disk, an external storage device, and the like.

Next, a position measurement method for measuring the position of the body tissue of the patient 100 by the ultrasonic waves according to the present embodiment will be described with reference to FIGS. 6 to 8.

First, an image collation method of the position measurement method according to the embodiment will be described with reference to FIG. 6. The processing is preferably performed by the ultrasonic measurement device 104, the sensor position measurement unit 105, and the first image construction unit 103A and the three-dimensional information ultrasonic image collation unit 103B of the target position calculation device 103.

First, the process is started (step S601). Here, it is assumed that the patient 100 is fixed to the bed and the ultrasonic sensor 104C is prepared on the body surface of the patient 100. Further, it is assumed that the three-dimensional information such as a three-dimensional image of the patient 100 is acquired.

Next, an ultrasonic signal is transmitted from the ultrasonic sensor 104C toward the body of the patient 100, and an ultrasonic reception signal returning back from the body of the patient 100 is collected by the ultrasonic sensor 104C (step S602). In synchronization with the collection of the ultrasonic signal, the sensor position measurement unit 105 measures a three-dimensional position of the ultrasonic sensor 104C (step S603).

Thereafter, the first image construction unit 103A constructs the ultrasonic image (first image) according to the ultrasonic signal collected in step S602 and the ultrasonic sensor position information measured in step S603 (step S606) .

In parallel with step S606, the three-dimensional information ultrasonic image collation unit 103B acquires three-dimensional information such as three-dimensional images from the three-dimensional information database 102 stored for each patient 100 and for each time series (step S604) . Thereafter, the three-dimensional information ultrasonic image collation unit 103B acquires the two-dimensional cross-sectional image according to the three-dimensional information corresponding to the ultrasonic image constructed in step S606, on the basis of the measured ultrasonic sensor position information (step S605).

After the processing in steps S605 and S606, both the two-dimensional cross-sectional image acquired in step S605 and the ultrasonic image constructed in step S606 are displayed in parallel in the display unit 106 and the like (step S607) .

Then, the image collation processing ends, and the processing proceeds to sound velocity calculation processing as shown in FIG. 7 (step S608).

Next, the sound velocity calculation method used by the position measurement device of the present embodiment will be described with reference to FIG. 7. The processing is preferably performed by the sound velocity calculation unit 103C of the target position calculation device 103.

First, the process is started (step S701). Here, it is assumed that the image collation in step S607 and the steps before are completed.

Next, the sound velocity calculation unit 103C extracts a boundary of the body tissue in the constructed ultrasonic image (step S702). The sound velocity calculation unit 103C extracts a boundary of the body tissue in the two-dimensional cross-sectional image that is collated according to the three-dimensional information (step S703). At this time, it is desirable to match the resolutions of the ultrasonic image constructed in step S606 and the two-dimensional cross-sectional image by adjusting imaging areas, pixel sizes, and luminance thereof. The method for matching the resolutions is as described above.

Then, the sound velocity calculation unit 103C calculates a position error between the boundary surface of the body tissue in the ultrasonic image extracted in step S702 and the boundary surface of the body tissue in the two-dimensional cross-sectional image extracted in step S703 (step S704) .

Next, the sound velocity calculation unit 103C calculates the sound velocities for each body tissue according to the position error calculated in step S704 (step S705).

Thereafter, the sound velocity calculation unit 103C constructs the ultrasonic image using the sound velocities of each body tissue calculated in step S705 (step S706).

Further, the sound velocity calculation unit 103C extracts a boundary of the body tissue in the ultrasonic image imaged in step S706 (step S707).

Thereafter, the sound velocity calculation unit 103C calculates an error between the position of the boundary of the body tissue imaged in step S707 and the position of the boundary of the body tissue extracted based on the three-dimensional information in step S703 (S708).

Next, the sound velocity calculation unit 103C determines whether the error calculated in step S708 is less than or equal to a threshold value set in advance (step S709) . If it is determined that the calculated error is less than or equal to the preset threshold value, it is assumed that the sound velocities calculated in step S705 satisfies the desired accuracy. The processing proceeds to store the sound velocities calculated above into the sound velocity database 103D for each body tissue, and then the processing ends (step S711) .

In contrast, if it is determined in step S709 that the error is larger than the threshold value set in advance, the processing returns back to step S704, and the sound velocity calculation processing is repeated until accuracy of the sound velocities of each body tissue is higher than a predetermined level.

Steps S701 to S711 correspond to the steps of collating at least one of the position, shape, and boundary of the body tissue in the first image and in the two-dimensional cross-sectional image to calculate the sound velocities of each body tissue of the patient 100.

Next, a second image construction method for constructing an image with reference to the sound velocity in the position measurement device of the present embodiment will be described with reference to FIG. 8. The processing is preferably performed by the second image construction unit 103E, the target position calculation unit 103F, and the target position output unit 103G of the target position calculation device 103.

First, the process is started (step S801). Here, it is assumed that the sound velocity calculation is completed, and the sound velocities of each body tissue of each patient are stored in the sound velocity database 103D.

Next, the second image construction unit 103E reads sound velocity data for each body tissue specific to the target patient 100 from the sound velocity database 103D (step S802) .

In parallel with or in advance of the above, an ultrasonic signal is transmitted toward the body of the patient 100 by the ultrasonic sensor 104C, and an ultrasonic reception signal returning back from the body of the patient 100 is collected by the ultrasonic sensor 104C (step S803) . In synchronization with the collection of the ultrasonic signal, the sensor position measurement unit 105 measures the three-dimensional position of the ultrasonic sensor 104C (step S804).

Thereafter, the second image construction unit 103E constructs, using the sound velocities read in step S802, the ultrasonic image (second image) according to the ultrasonic signal collected in step S803 and the ultrasonic sensor position measured in step S804 (step S805).

Next, the second image construction unit 103E outputs the ultrasonic image constructed in step S805 to the target position calculation unit 103F (step S806).

Thereafter, the target position calculation unit 103F calculates the three-dimensional position of the target using the output ultrasonic image, and outputs the calculated three-dimensional position information to the display unit 106 via the target position output unit 103G (step S807).

Finally, the processing ends (step S808).

Next, effects of the present embodiment will be described.

The position measurement device for measuring the position of the body tissue of the patient 100 by the ultrasonic waves according to the first embodiment of the present invention includes the ultrasonic sensor 104C, the sensor position measurement unit 105 for measuring the position of the ultrasonic sensor 104C, and the target position calculation device 103 that constructs the ultrasonic image according to the ultrasonic waveform acquired by the ultrasonic sensor 104C. The target position calculation device 103 constructs the first image according to the ultrasonic waveform acquired by the ultrasonic sensor 104C, collates the three-dimensional image acquired in advance and the first image on the basis of the sensor position information acquired by the sensor position measurement unit 105, calculates the sound velocities for each body tissue of the patient 100, constructs the second image according to the ultrasonic waveform acquired by the ultrasonic sensor 104C using the calculated sound velocities of each body tissue of the patient 100, and calculates the position of the target tissue according to the second image.

With such a configuration, the sound velocities for each patient and each tissue thereof can be calculated in advance. Therefore, it is possible to construct an ultrasonic image in which the actual state of the patient body is reflected more accurately than that in the related art, during the treatment. In this way, it is possible to calculate the target position in the patient body with high accuracy using the ultrasonic image that can accurately depict soft tissues with low invasiveness, and it is possible to measure the position of the target object in the body tissue with higher accuracy than that of the related art.

The three-dimensional information ultrasonic image collation unit 103B of the target position calculation device 103 can improve the accuracy of the collation between the three-dimensional information image and the actual ultrasonic image by adjusting imaging areas, pixel sizes, and luminance of the first image and the three-dimensional image to match the resolutions of the three-dimensional image and the first image. Therefore, the amount of deviation between the two images can be calculated more accurately, and the accuracy of the sound velocities to be calculated can be further improved.

Further, the three-dimensional information ultrasonic image collation unit 103B of the target position calculation device 103 can quickly and easily match the resolutions of the three-dimensional information image and the actual ultrasonic image by matching the resolutions using the feature points in the first image and the three-dimensional image.

Further, the sound velocity calculation unit 103C reconstructs the first image according to the calculated sound velocities and continues reconstructing the reconstructed image until the difference between the three-dimensional image acquired in advance and the reconstructed first image is equal to or less than the predetermined value. In this way, the accuracy of the sound velocities for each patient and each body tissue thereof can be increased, and the ultrasonic image can be rendered more accurately.

Further, since the sound velocity database 103D that stores the sound velocities of each body tissue of the patient 100 calculated by the target position calculation device 103 is further provided, it is not necessary to calculate the sound velocities for each patient and each body tissue thereof during treatment, and it is possible to more easily construct the ultrasonic image reflecting the actual state of the patient body during treatment.

In the invention, in order to calculate the target position in the patient body with high accuracy, it is most important to obtain the ultrasonic image that can accurately depict the soft tissues with low invasiveness, so that it is most effective to collate the three-dimensional image acquired in advance and the first image on the basis of the sensor position information acquired by the sensor position measurement unit to calculate the sound velocities for each body tissue of the patient 100 and preferably store the calculated velocities in a database.

That is, it is most effective to provide the first image construction unit 103A, the three-dimensional information ultrasonic image collation unit 103B, the sound velocity calculation unit 103C, and the sound velocity database 103D.

Therefore, by incorporating configurations corresponding to the first image construction unit 103A to the sound velocity calculation unit 103C into an existing ultrasonic examination device and the like, an ultrasonic image in which the actual state of the patient 100 is reflected more accurately can be obtained even in the existing device. Further, it is more desirable to incorporate a configuration corresponding to the sound velocity database 103D.

### <Second Embodiment>

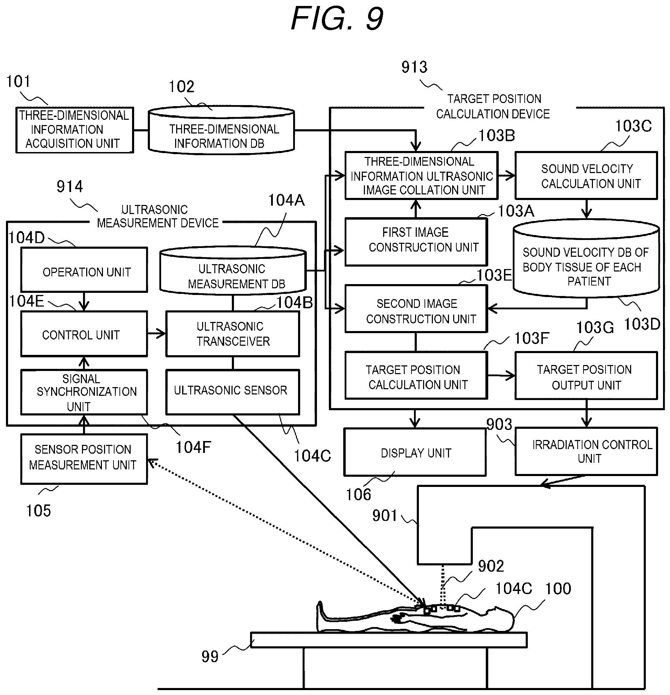

A treatment system including a position measurement device according to a second embodiment of the invention will be described with reference to FIG. 9. The same components as in the first embodiment are denoted by the same reference numerals, and the description thereof is omitted. The same applies to the following embodiments.

FIG. 9 is a conceptual diagram of a radiation system including the position measurement device according to the second embodiment.

The treatment system according to the present embodiment shown in FIG. 9 specifies a target body tissue position, irradiates a treatment target site (target) with therapeutic radiation on the basis of the specified body tissue position, and includes the position measurement device described in the first embodiment, a radiation irradiation device 901 that irradiates the target with radiation and an irradiation control unit 903 that controls a radiation irradiation position in the radiation irradiation device 901 on the basis of the body tissue position measured using the position measurement device.

The therapeutic radiation to be used includes: a proton beam; a heavy particle beam of carbon, helium, and the like; an X-ray, a neutron beam, and the like, and the type thereof is not particularly limited.

The irradiation control unit 903 receives the target position calculated by a target position calculation device 913 as a signal from a target position output unit 103G in the target position calculation device 913, and controls the irradiation position of radiation 902 of X-rays or particle beams to be irradiated on a patient 100 by controlling the radiation irradiation device 901. As a result, the radiation irradiation position is concentrated in a region of a planned treatment target site in treatment planning and is irradiated with radiation.

The target position calculation device 913 of the position measurement device has the same configuration as the target position calculation device 103 described in the first embodiment.

In the present embodiment, by monitoring a respiration state of the patient 100 when the radiation is irradiated, it is also possible to specify an appropriate timing at which the treatment target site passes through the region in target coordinates so as to start or stop the irradiation of the radiation.

Further, by repeatedly performing the target position calculation at an appropriate frame rate, the radiation 902 can be controlled according to the movement of the treatment target site.

Therefore, in the present embodiment, an ultrasonic measurement device 914 of the position measurement device includes an operation unit 104D, a control unit 104E, and a signal synchronization unit 104F in addition to the units of the ultrasonic measurement device 104 described in the first embodiment.

The signal synchronization unit 104F is a part that calculates a respiratory phase by using position information of an ultrasonic sensor 104C measured by a sensor position measurement unit 105 and that monitors a respiration state of the patient.

The control unit 104E specifies an appropriate timing at which the treatment target site passes through the region in the target coordinates on the basis of the respiration state of the patient monitored by the signal synchronization unit 104F, and outputs the specified result to the irradiation control unit 903 via the target position calculation device 913. The irradiation control unit 903 can execute control to start or stop irradiation of radiation according to the specified result.

Further, the control unit 104E can calculate an appropriate frame rate according to the movement of the treatment target site based on the respiration state of the patient monitored by the signal synchronization unit 104F. The control unit 104E outputs the calculated frame rate to the ultrasonic sensor 104C via an ultrasonic transceiver 104B, and transmits and receives ultrasonic waves for each appropriate frame rate.

The operation unit 104D is a part for an operator to input a frame rate for ultrasonic transmission and reception. When an item input by the operation unit 104D exists, the control unit 104E causes the transmission and reception of ultrasonic waves to be performed using the existing information.

Other components and operations of the position measurement device are substantially the same as those of the position measurement device of the first embodiment, and detailed description thereof is omitted.

In the treatment system of the second embodiment of the invention, since the position measurement device of the first embodiment described above is provided, the target position of the body can be measured with low invasiveness and higher accuracy than the related art. Therefore, the radiation irradiation position can be accurately controlled to irradiate the treatment target site of the patient with radiation with high accuracy.

### <Third Embodiment>

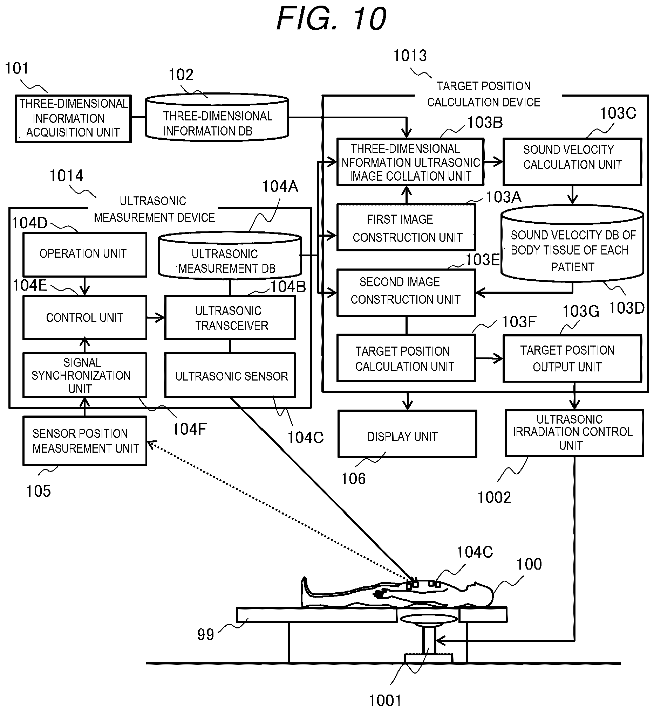

A treatment system including a position measurement device according to a third embodiment of the invention will be described with reference to FIG. 10.

FIG. 10 is a conceptual diagram of an ultrasonic treatment system including the position measurement device according to the third embodiment.

The treatment system according to the present embodiment shown in FIG. 10 specifies a target body tissue position, irradiates a treatment target site (target) with therapeutic ultrasonic waves on the basis of the specified body tissue position, and includes the position measurement device described in the first embodiment, an ultrasonic irradiation device 1001 that irradiates the target with ultrasonic waves, and an ultrasonic irradiation control unit 1002 that controls an ultrasonic irradiation position in the ultrasonic irradiation device 1001 on the basis of the body tissue position measured using the position measurement device.

The ultrasonic irradiation control unit 1002 receives the target position calculated by a target position calculation device 1013 as a signal from a target position output unit 103G in the target position calculation device 1013, and controls the ultrasonic irradiation position on a patient 100 by controlling the ultrasonic irradiation device 1001. As a result, the ultrasonic irradiation position is concentrated in a region of a planned treatment target site in treatment planning and is irradiated with ultrasonic waves.

The target position calculation device 1013 of the position measurement device of the present embodiment has the same configuration as the target position calculation device 103 described in the first embodiment and the target position calculation device 913 described in the second embodiment. An ultrasonic measurement device 1014 has the same configuration as the ultrasonic measurement device 914 described in the second embodiment.

Other components and operations of the position measurement device are substantially the same as those of the position measurement device of the first embodiment, and detailed description thereof is omitted.

In the treatment system of the third embodiment of the invention, since the position measurement device of the first embodiment described above is provided, the target position of the body can be measured with low invasiveness and higher accuracy than the related art. Therefore, the ultrasonic irradiation position can be accurately controlled to irradiate the treatment target site of the patient with ultrasonic waves with high accuracy.

### <Fourth Embodiment>

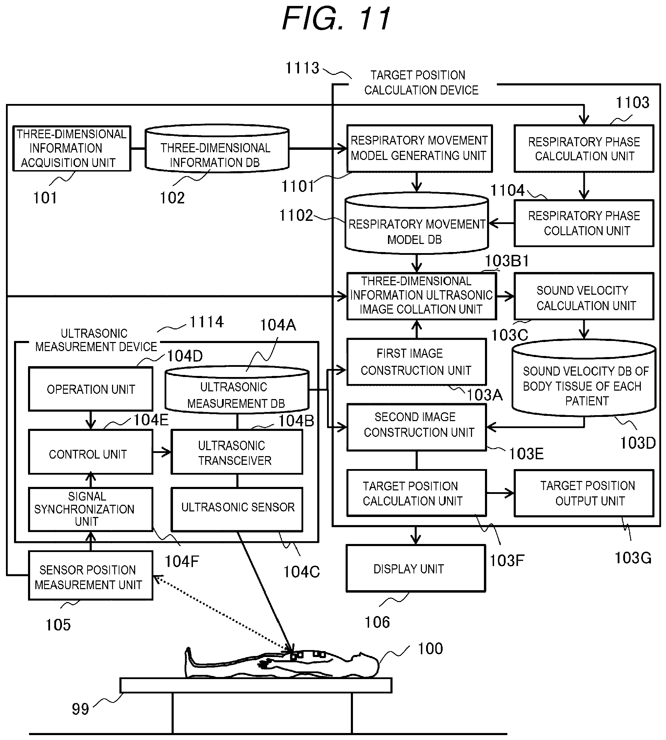

A position measurement device and a position measurement method according to a fourth embodiment of the invention will be described with reference to FIG. 11.

FIG. 11 is a conceptual diagram of the position measurement device according to the fourth embodiment.

The position measurement device of the present embodiment shown in FIG. 11 detects respiratory phases of a patient, collates the respiratory phases, and calculates a target position in the body.

As shown in FIG. 11, a target position calculation device 1113 of the position measurement device further includes a respiratory movement model generating unit 1101, a respiratory movement model database 1102, a respiratory phase calculation unit 1103, and a respiratory phase collation unit 1104, in addition to the units of the target position calculation device 103 described in the first embodiment.

The respiratory movement model generating unit 1101 generates a respiratory movement model of a patient 100 according to a three-dimensional image which is acquired in the plurality of respiratory phases and which is stored in a three-dimensional information database 102, and stores the model in the respiratory movement model database 1102. The respiratory movement model is, for example, time-series data corresponding to one breath of the patient 100.

The respiratory phase calculation unit 1103 calculates the respiratory phases of the patient according to position information of an ultrasonic sensor 104C measured by a sensor position measurement unit 105.

On the basis of the respiratory phases of the patient calculated by the respiratory phase calculation unit 1103, the respiratory phase collation unit 1104 transmits, to the respiratory movement model database 1102, respiratory phase information corresponding to the phases of the respiration divided in time series in the respiratory movement model.

A three-dimensional information ultrasonic image collation unit 103B1 selects patient position information indicating a corresponding respiratory phase according to the respiratory movement model stored in the respiratory movement model database 1102, and constructs a cross-sectional image to be collated with an acquired ultrasonic image (first image) .

An ultrasonic measurement device 1114 has the same configuration as the ultrasonic measurement device 914 described in the second embodiment and the ultrasonic measurement device 1014 described in the third embodiment, and may also have the same configuration as the ultrasonic measurement device 104 described in the first embodiment.

Other components and operations are substantially the same as those of the position measurement device and the position measurement method of the first embodiment described above, and detailed description thereof is omitted.

The position measurement device and the position measurement method according to the fourth embodiment of the invention provide substantially the same effects as those of the position measurement device and the position measurement method according to the first embodiment described above.

Further, the target position calculation device 1103 can generate the respiratory movement model on the basis of the three-dimensional image acquired in the plurality of respiratory phases, calculate the respiratory phases by using the position information of the ultrasonic sensor 104C measured by the sensor position measurement unit 105, and selects the three-dimensional image to be collated with the first image on the basis of the calculated respiratory phases, and thus the three-dimensional information image and the actual ultrasonic image can be collated with higher accuracy and higher speed. Therefore, the time required to measure the position with high accuracy can be further shorten.

The ultrasonic position measurement device of the present embodiment can be applied to the treatment systems as shown in FIGS. 9 and 10.

### <Fifth Embodiment>

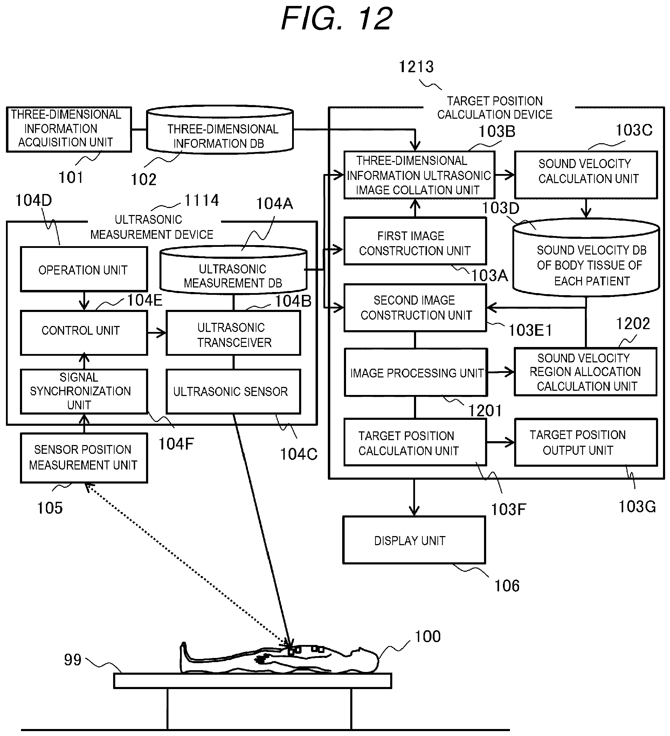

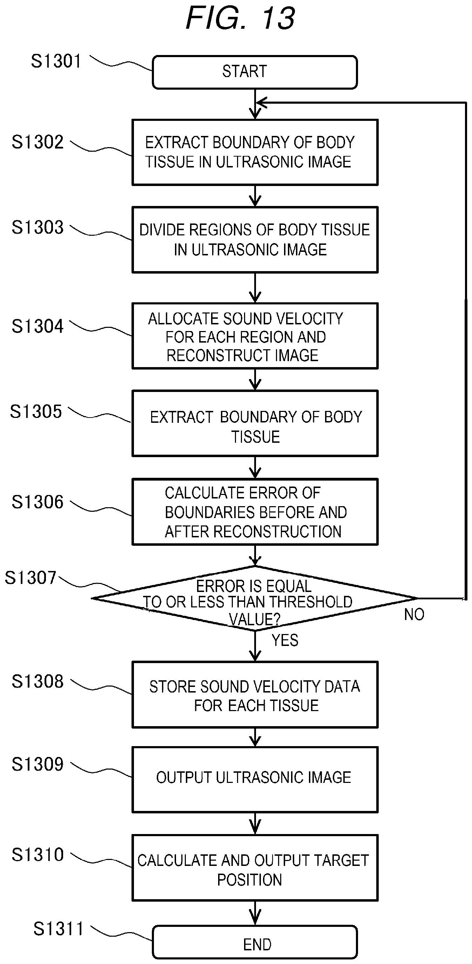

A position measurement device and a position measurement method according to a fifth embodiment of the invention will be described with reference to FIGS. 12 and 13.

FIG. 12 is a conceptual diagram of a position measurement device according to the fifth embodiment. FIG. 13 is a flowchart showing an example of a reconstruction method for an ultrasonic image executed by the position measurement device according to the fifth embodiment.

The position measurement device of the present embodiment shown in FIG. 12 includes a target position calculation device 1213 instead of the target position calculation device 103 of the position measurement device described in the first embodiment.

The target position calculation device 1213 includes a second image construction unit 103E1 instead of the second image construction unit 103E among the configurations of the target position calculation device 103 described in the first embodiment. The target position calculation device 1213 further includes an image processing unit 1201 and a sound velocity region allocation calculation unit 1202.

The image processing unit 1201 of the target position calculation device 1213 acquires position information of the body tissue from a second image constructed by the second image construction unit 103E1. For example, a boundary of the body tissue is extracted from the second image.

The sound velocity region allocation calculation unit 1202 calculates regions of the body tissue to which sound velocity information stored in a sound velocity database 103D is allocated respectively to the second image according to the position information acquired by the image processing unit 1201, and allocates each piece of sound velocity data to the regions where the sound velocities were calculated in advance. The second image construction unit 103E1 reconstructs an ultrasonic image using the newly allocated sound velocity data.

The target position calculation device 1213 continuously constructs the reconstructed image until a difference between the reconstructed ultrasonic image and the second image is equal to or less than a predetermined value. Then, when the difference is equal to or less than the predetermined value, the reconstructed image is output to a target position calculation unit 103F as the second image. The target position calculation unit 103F calculates a position of a target tissue according to the output reconstructed image.

Next, a position measurement method for measuring a position of the body tissue of a patient 100 by ultrasonic waves according to the present embodiment will be described with reference to FIG. 13.

Here, an image collation method is substantially the same as that in FIG. 6, a sound velocity calculation method performed in advance before irradiation is substantially the same as that in FIG. 7, and thus detailed descriptions thereof are omitted.

A second image construction method for image constructing with reference to the sound velocities in the position measurement device of the present embodiment will be described below with reference to FIG. 13. The processing preferably performed in the second image construction unit 103E1, the image processing unit 1201, the sound velocity region allocation calculation unit 1202, the target position calculation unit 103F, and the target position output unit 103G of the target position calculation device 1213.

First, the process is started (step S1301). Here, it is assumed that sound velocities for each body tissue of each patient 100 are stored in the sound velocity database 103D, and an ultrasonic signal is acquired from an ultrasonic sensor 104C disposed on the patient 100.

Next, the sound velocity region allocation calculation unit 1202 extracts a plurality of parameters such as shapes, sizes, and positions of the body tissue in the ultrasonic image by image processing (step S1302).

Thereafter, the sound velocity region allocation calculation unit 1202 divides the regions of the body tissues in the ultrasonic image on the basis of the parameters extracted in advance (step S1303).

Next, the sound velocity region allocation calculation unit 1202 divides the sound velocity data stored in the sound velocity database 103D for each region divided in step S1303, and reconstructs an ultrasonic image (step S1304).

Next, the sound velocity region allocation calculation unit 1202 extracts a boundary of the body tissue in the ultrasonic image constructed in step S1304 (step S1305).

Thereafter, the sound velocity region allocation calculation unit 1202 calculates an error between the boundary positions of the body tissues of the ultrasonic image reconstructed in step S1304 and the ultrasonic image before reconstruction (step S1306).

Then, the sound velocity region allocation calculation unit 1202 determines whether the error calculated in step S1306 is less than or equal to an appropriately determined threshold value (step S1307). If it is determined that the threshold value is equal to or less than the threshold value, the processing proceeds to step S1308. In contrast, if it is determined that the error is larger than the threshold value, the processing returns back to step S1302, the error is calculated again, the sound velocity allocation region is corrected, and the image reconstruction is repeatedly performed until the error is equal to or less than the threshold value.

If it is determined in step S1307 that the error is equal to or less than the threshold value, the sound velocity region allocation calculation unit 1202 updates and stores the updated sound velocity data for each body tissue in the sound velocity database 103D (step S1308).

Next, the image processing unit 1201 outputs the corrected ultrasonic image to the target position calculation unit 103F (step S1309).

Thereafter, the target position calculation unit 103F and the target position output unit 103G perform position calculation using the ultrasonic image output in step S1309 (step S1310). Details of this step are the same as those in step S807 shown in FIG. 8.

Here, an ultrasonic measurement device 1214 has the same configuration as the ultrasonic measurement device 914 described in the second embodiment, the ultrasonic measurement device 1014 described in the third embodiment, and the ultrasonic measurement device 1114 described in the fourth embodiment, and can also have the same configuration as the ultrasonic measurement device 104 described in the first embodiment.

Other components and operations are substantially the same as those of the position measurement device and the position measurement method of the first embodiment described above, and detailed description thereof is omitted.

The position measurement device and the position measurement method according to the fifth embodiment of the invention provide substantially the same effects as those of the position measurement device and the position measurement method according to the first embodiment.

The target position calculation device 1213 acquires the position information of the body tissue in the second image, allocates the sound velocity information to the second image according to the acquired position information to reconstruct the image, continues constructing the reconstructed image until the difference between the reconstructed image and the second image is equal to or less than the predetermined value, and calculates the position of the target tissue using the reconstructed image as the second image when the difference is equal to or less than the predetermined value, so that the ultrasonic image reflecting the sound velocities of the body tissues at the time of actually measuring the position can be constructed with higher accuracy. Therefore, the position of the body tissue can be measured with higher accuracy.

In the present embodiment, respiratory phases of the patient can be detected, and the respiratory phases can be collated to calculate the target position in the body, as in the fourth embodiment. Further, the ultrasonic position measurement device of the present embodiment can be applied to the treatment systems as shown in FIGS. 9 and 10.

### <Others>

The invention is not limited to the above embodiments, and may include various modifications. The embodiments described above are detailed for easy understanding of the invention but the invention is not necessarily limited to those including all the above configurations.

Further, a part of the configurations of one embodiment can be replaced with a configuration of another embodiment, and the configuration of one embodiment can be added to the configuration of another embodiment. In addition, it is possible to add, remove, and replace other configurations to, from and with a part of the configurations of each embodiment.

## Claims

1. A position measurement device configured to measure a position of a body tissue of a patient by ultrasonic waves, comprising:
an ultrasonic sensor;
a sensor position measurement unit configured to measure a position of the ultrasonic sensor; and
a position calculation device configured to construct an ultrasonic image according to an ultrasonic waveform acquired by the ultrasonic sensor; wherein
the position calculation device is configured to:
construct a first image according to the ultrasonic waveform acquired by the ultrasonic sensor, collate a three-dimensional image acquired in advance with the first image on the basis of sensor position information acquired by the sensor position measurement unit to calculate sound velocities of each body tissue of the patient, and
construct a second image according to the ultrasonic waveform acquired by the ultrasonic sensor using the calculated sound velocities of each body tissue of the patient so as to calculate a position of a target tissue according to the second image.

2. The position measurement device according to claim 1, wherein
the position calculation device is configured to:
generate a respiratory movement model on the basis of three-dimensional images acquired in a plurality of respiratory phases, and
calculate a respiratory phase by using the position information of the ultrasonic sensor measured by the sensor position measurement unit so as to select, from the respiratory movement model, a three-dimensional image to be collated with the first image on the basis of the calculated respiratory phase.

3. The position measurement device according to claim 1, wherein
the position calculation device is configured to acquire position information of the body tissue in the second image, constructs a reconstructed image by allocating sound velocity information to the second image according to the acquired position information, construct the reconstructed image until a difference between the reconstructed image and the second image is equal to or less than a predetermined value, and calculate a position of the target tissue using the reconstructed image obtained when the difference is equal to or less than the predetermined value as the second image.

4. The position measurement device according to claim 1, wherein
the position calculation device is configured to:
reconstruct the first image according to the calculated sound velocities at the time of calculating the sound velocities of each body tissue of the patient, construct the reconstructed image until a difference between the three-dimensional image acquired in advance and the reconstructed first image is equal to or less than a predetermined value, and set the sound velocities obtained when the difference is equal to or less than the predetermined value as the sound velocities of each body tissue of the patient.

5. The position measurement device according to claim 1, wherein
the position calculation device is configured to adjust an imaging area, a pixel size, and luminance of the first image or the three-dimensional image to match image resolutions of the three-dimensional image and the first image.

6. The position measurement device according to claim 4, wherein
the position calculation device is configured to match the resolutions using a feature point in the first image and the three-dimensional image.

7. The position measurement device according to claim 1 further comprising:
a sound velocity database configured to store the sound velocities of each body tissue of the patient calculated by the position calculation device.

8. A treatment system configured to specify a target body tissue position and perform treatment on the basis of the specified body tissue position, comprising:
the position measurement device according to claim 1;
a radiation irradiation device configured to irradiate a target with radiation; and
a radiation control unit configured to control a radiation irradiation position in the radiation irradiation device on the basis of the body tissue position measured by the position measurement device.

9. A treatment system configured to specify a target body tissue position and perform treatment on the basis of the specified body tissue position, comprising:
the position measurement device according to claim 1;
an ultrasonic irradiation device configured to irradiate a target with ultrasonic waves; and
an ultrasonic control unit configured to control an ultrasonic irradiation position in the ultrasonic irradiation device on the basis of the body tissue position measured by the position measurement device.

10. A position measurement method for measuring a position of a body tissue of a patient by ultrasonic waves, comprising:
a step of acquiring three-dimensional information of the patient;
a step of transmitting ultrasonic waves toward a patient body and receiving ultrasonic waves returning back from the patient body;
a step of measuring a position of an ultrasonic sensor configured to transmit and receive the ultrasonic waves;
a step of constructing a first image according to position information of the ultrasonic sensor and a received ultrasonic waveform;
a step of acquiring a two-dimensional cross-sectional image corresponding to the first image constructed according to the three-dimensional information, on the basis of the position information of the ultrasonic sensor;
a step of calculating sound velocities of each body tissue of the patient by collating at least one of positions, shapes, and boundaries of body tissues of the first image and the two-dimensional cross-sectional image;
a step of constructing a second image using the calculated sound velocities; and
a step of calculating a position of a target tissue according to the second image.

11. The position measurement method according to claim 10, further comprising:
a step of acquiring the three-dimensional information in a plurality of respiratory phases;
a step of generating a respiratory movement model on the basis of the three-dimensional information; and
a step of calculating a respiratory phase by using the position information of the ultrasonic sensor, wherein
in the step of acquiring the two-dimensional cross-sectional image, a corresponding two-dimensional cross-sectional image is acquired by selecting, from the respiratory movement model, three-dimensional information to be collated with the first image on the basis of the calculated respiratory phase.

12. The position measurement method according to claim 10, further comprising:
a step of acquiring position information of a body tissue in the second image constructed by using the calculated sound velocities; and
a step of allocating sound velocity information to the second image according to the acquired position information to construct a reconstructed image; wherein
in the step of constructing the second image, the reconstructed image is constructed until a difference between the reconstructed image and the second image is equal to or less than a predetermined value, and the reconstructed image obtained when the difference is equal to or less than the predetermined value is set as the second image.

13. The position measurement method according to claim 10, wherein
in the step of calculating the sound velocities of each body tissue of the patient, the first image is reconstructed according to the calculated sound velocities, the reconstructed image is constructed until a difference between the three-dimensional image acquired in advance and the reconstructed first image is equal to or less than a predetermined value, and the sound velocities obtained when the difference is equal to or less than the predetermined value are set as the sound velocities of each body tissue of the patient.

14. The position measurement method according to claim 10, wherein
in the step of calculating the sound velocities of each body tissue of the patient, an imaging area, a pixel size, and luminance of the first image or the three-dimensional image are adjusted to match image resolutions of the three-dimensional image and the first image.
